# EUROPÄISCHE PATENTSCHRIFT

(11) **EP 0 934 758 B1**
(45) Veröffentlichungstag und Bekanntmachung des Hinweises auf die Patenterteilung: **27.07.2005**
(21) Anmeldenummer: 98250442.5
(22) Anmeldetag: 17.12.1998
(51) Int. Cl.: A61N 1/368

(54) **Herzstimulator**
Cardiac Stimulator
Stimulateur cardiaque

(30) Priorität: 17.12.1997 DE 19758109
(43) Veröffentlichungstag der Anmeldung: 11.08.1999
(73) Patentinhaber: BIOTRONIK Mess- und Therapiegeräte GmbH & Co Ingenieurbüro Berlin, 12359 Berlin (DE)
(72) Erfinder: Lang, Volker, 91074 Herzogenaurach (DE)
(74) Vertreter: von Oppen, Joachim, Dipl.-Ing.

(56) Entgegenhaltungen:
- EP-A- 0 607 951
- US-A- 4 305 396
- US-A- 4 556 062
- US-A- 5 534 016

## Beschreibung

Die Erfindung betrifft eine Herzstimulatoranordnung mit einem Stimulationsimpulsgenerator und mit diesem ver-bundenen Ausgangsmitteln zur Abgabe von Stimulationsimpulsen an den Ventrikel eines Herzens, einer Herzsignal-Eingangsstufe zur Erfassung evozierter Herzsignale, insbesondere der ventrikulären evozierten Reizantwort VER, einer ausgangsseitig mit dem Stimulationsimpulsgenerator verbundenen AV-Verzögerungseinheit zur Erzeugung ei-nes AV-Intervalls zwischen einem atriellen Herzereignis oder atriellen Stimulationsimpuls und einem an den Ventrikel abgegebenen Stimulationsimpuls, einer eingangsseitig mit der Herzsignal-Eingangsstufe und ausgangsseitig mit der AV-Verzögerungseinheit verbundenen AV-Intervalt-Berechnungseinrichtung zur Berechnung des AV-Intervalls aufgrund mindestens eines Parameters des Herzsignals.

Ratenadaptive Herzschrittmacher, bei denen die Stimulationsfrequenz bzw. -rate in Abhängigkeit von im Körper des Patienten aufgenommenen Signalen eingestellt wird, die den physiologischen Bedarf des Patienten in Bezug auf die Herzaktivität reflektieren, sind seit Jahren in vielgestaltigen Ausführungen bekannt und im klinischen Einsatz. Einen Überblick über die mit der Entwicklung der Ratenadaption in der Schrittmachertechnik verfolgten Ziele und die eingeschlagenen Wege gibt K. Stangl et al.: Frequenzadaptive Herzschrittmacher, Darmstadt 1990.

Zu den bekannten ratenadaptiven Schrittmachern zählt der sogenannte QT-Schrittmacher, bei dem die Einstellung der Stimulationsrate anhand der Zeitspanne zwischen einem Stimulationsimpuls und dem Auftreten der T-Zacke des durch den Stimulus initiierten (evozierten) ventrikulären Herzaktionssignals, des sogenannten QT-Intervalls, erfolgt (a.a.O. Seiten 255-272). Dieses Zeitintervall kann zur Ratensteuerung eines Herzschrittmachers herangezogen werden, weil es sowohl von der Herzfrequenz als auch von der Belastung abhängt und die Abhängigkeit von der Herzfrequenz zumindest in einem bestimmten Zeitbereich annähernd linear ist.

Gemäß EP 0 232 528 B1 und EP 0 237 767 B1 erfolgt die Steuerung der Stimulationsrate aufgrund eines Vergleiches eines integrierten Herzaktionspotentials mit einem vorab bestimmten Ziel- bzw. Vergleichswert; im Grunde findet hierbei eine Auswertung der Zeitdauer vom Stimulus bis zum Ende der R-Zacke des ventrikulären EKG ("Depolarisationsgradientendauer") statt.

Eine die physiologische Effizienz der Stimulation bei Zweikammerschrittmachern wesentlich beeinflussende Größe ist (neben der Stimulationsrate) das AV-Intervall zwischen den an das Atrium und an den Ventrikel abgegebenen Stimulationsimpulsen. Während dieses Intervall bei frühen, starrfrequent arbeitenden Zweikammerschrittmachern konstant eingestellt und später programmierbar war, ist die feste Einstellung auf einen konstanten oder programmierbaren Wert bei einem ratenadaptiven Zweikammerschrittmacher hämodynamisch unvorteilhaft. Es wurde daher eine Reihe von Möglichkeiten zur selbsttätigen AV-Intervallanpassung während des Schrittmacherbetriebs vorgeschlagen; vgl. EP 0 494 487 A2 oer EP 0 596 598 A2.

Aus EP 0 607 951 A2 ist ein Verfahren zur Optimierung des AV-Intervalls bekannt, bei dem das AV-Intervall bei der minimal zulässigen Stimulationsrate über einen vorbestimmten Wertebereich variiert und das jeweils zugehörige QT-Intervall gemessen wird. Als optimaler Wert des AV-Intervalls wird dann derjenige eingestellt, bei dem das QT-Intervall maximal ist.

Der in US 5 534 016 beschriebene Schrittmacher nutzt die Abhängigkeit der Amplitude der T-Welle der evozierten Reizantwort vom eingestellten AV-Intervall zur Feinjustierung des AV-Intervalls aus.

Der Erfindung liegt die Aufgabe zugrunde, einen Herzstimulator der eingangs genannten Gattung bereitzustellen, bei dem das AV-Intervall auf besonders zuverlässige und störsichere Weise eingestellt wird.

Die Aufgabe wird durch eine Herzstimulator anordnung der eingangs genannten Art gelöst bei der
die Herzsignal-Eingangsstufe zur Signalformerfassung evozierter Herzsignale, der Morphologie der ventrikulären evozierten Reizantwort VER ausgebildet ist und dass Mittel (125.4) zur Erfassung der Maximal-Amplitude (R-max) der R--Welle und/oder der Position und Amplitude der Maxima der R+-Welle und der T+-Welle der VER vorgesehen sind, und
die AV-Intervall-Berechnungseinrichtung zur Berechnung des AV-Intervalls aufgrund der Maximalamplitude der R--Welle und/oder der Position und Amplitude der Maxima der R+-Welle und der T+-Welle ausgebildet ist.

Die Erfindung schließt die technische Lehre ein, charakteristische und auch durch Störungen, Nulliniendrift etc. nicht wesentlich verfälschte belastungsabhängige Änderungen der Signalmorphologie eines evozierten Herzsignals zur Steuerung des AV-Intervalls heranzuziehen. Hierfür besonders geeignete Herzsignalkomponenten stellen die R-- und die R+-Welle sowie die T-Welle der ventrikulären evozierten Reizantwort (VER) dar.

Der Herzsignal-Eingangsstufe können in einer Ausführungsform Mittel zur Erfassung der Maximalamplitude der R--Welle und/oder der Position und Amplitude der Maxima der R+-Welle und der T+-Welle der VER zugeordnet sein, und die AV-Intervall-Berechungseinrichtung kann zur Berechnung des AV-Intervalls aufgrund der Maximalamplitude der R--Welle und/oder der Position und Amplitude der Maxima der R+-Welle und der T+-Welle ausgebildet sein. Diese Ausführungsform zeichnet sich durch hohe Genauigkeit der Auswertung der Herzsignalmorphologie aus.

In einer alternativen Ausführung umfaßt die Herzsignal-Eingangsstufe Mittel zur Erfassung der Maximalamplitude der R--Welle und/oder einen Zeitgeber zur Vorgabe je eines Amplitudenwert-Erfassungszeitpunktes und Mittel zur Erfassung der Amplitudenwerte zum jeweiligen Erfassungszeitpunkt im Bereich der R+-Welle und der T+-Welle der VER, und die AV-Intervall-Berechungseinrichtung ist zur Berechnung des AV-Intervalls aufgrund der Maximalamplitude der R--Welle. und/oder der Amplitudenwerte im Bereich der R+-Welle und der T+-Welle ausgebildet. Diese Ausführung ist einfacher und stromsparend zu realisieren, da die Amplitudenwerte der R+- bzw. T--Welle zu vorgegebenen Zeitpunkten nach dem Stimulusimpuls ausgewertet werden und die rechentechnisch aufwendige Suche nach den jeweiligen lokalen Maxima hierbei nicht erforderlich ist. Die Erfassungszeitpunkte werden im einfachsten Fall invariant bei der Variation des AV-Intervalls und/oder gegenüber Änderungen der Stimulationsrate gehalten.

Die AV-Intervall-Berechnungseinrichtung umfaßt in bevorzugter Ausführung arithmetische Mittel zur Bestimmung der Steigung - genauer: des Betrages der Steigung - der VER zwischen den Maxima bzw. den Amplitudenwert-Erfassungszeitpunkt-Wertepaaren der R+-Welle und der T+-Welle, die wahlweise als relevante Größe zur Optimierung des AV-Intervalls herangezogen wird. Alternativ hierzu oder zugleich kann der Betrag des Amplitudenwertes der R--Welle ausgewertet werden.

Die Ermittlung des physiologisch optimalen AV-Intervalls erfolgt in Einstellphasen bzw. -zyklen, in denen bei festgehaltener Stimulationsrate ein vorgegebener Wertebereich von AV-Intervallwerten durchlaufen und derjenige AV-Intervallwert als optimal festgehalten wird, bei dem ein sich auf die Signalmorphologie beziehendes Bewertungskriterium am ehesten erfüllt ist. Dies kann insbesondere dasjenige AV-Intervall sein, bei dem der Betrag der Steigung der VER zwischen den Maxima bzw. den Amplitudenwert-Erfassungszeitpunkt-Wertepaaren der R+-Welle und der T+-Welle und/oder der Amplitudenwert der R--Welle maximal ist.

Die AV-Intervall-Berechnungseinrichtung oder die AV-Verzögerungseinheit selbst ist bevorzugt zur Ausgabe eines AV-Intervall-Ersatzwertes im Falle der Nichtverfügbarkeit eines aktuell berechneten AV-Intervalls, speziell bei Inhibierung des Stimulationsimpulsgenerators bzw. Nichterfassung eines evozierten Herzsignals oder für den Fall, daß der Patient über längere Zeit keinen zur Messung geeigneten Ruhzustand einnimmt, ausgebildet. Dies ist zweckmäßig, weil die Durchführung eines Kalibrationszyklus zur Berechnung der physiologisch optimalen AV-Intervalle das Vorliegen einer evozierten Reizantwort sowie möglichst einen liegenden Zustand mit niedriger Belastung des Patienten voraussetzt und daher bei inhibiertem Schrittmacherbetrieb und/oder anhaltend hoher Belastung nicht möglich ist.

Vorteilhafte Weiterbildungen der Erfindung sind im übrigen in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: eine Darstellung zur Auswertung der Signalmorphologie des VER für die AV-Intervall-Steuerung gemäß einer Ausführungsform der Erfindung,
- Figur 2: eine Darstellung zur Auswertung der Signalmorphologie des VER für die AV-Intervall-Steuerung gemäß einer weiteren Ausführungsform,
- Figur 3: eine Darstellung des Zusammenhangs zwischen AV-Intervall und Stimulationsfrequenz bei einer Ausführungsform der Erfindung,
- Figur 4: ein Funktions-Blockschaltbild wesentlicher Komponenten eines ratenadaptiven Zweikammerschrittmachers gemäß einer Ausführungsform und
- Figur 5: eine Detaildarstellung des Schrittmachers gemäß Fig 4.

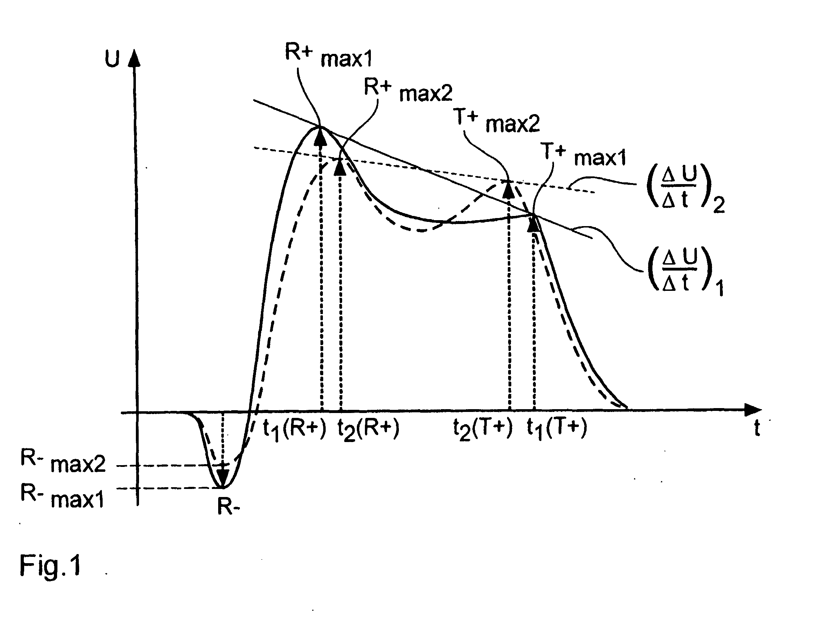

Fig. 1 ist eine schematische grafische Darstellung der R--Welle, der R+-Welle und der T+-Welle zweier bei konstanter Stimulationsfrequenz und verschiedenen AV-Intervallwerten aufgenommener VER-Signale VER₁ und VER₂; es ist die Signalspannung U über der Zeit t ab dem Stimulusimpuls aufgetragen. An den Kurven ist zu erkennen, daß sich die maximalen Signalspannungen R-ₘₐₓ₁, R-ₘₐₓ₂ im R--Abschnitt ebenso unterscheiden wie Lage und Spannungswert der Amplitudenmaxima im R+- sowie im T+-Abschnitt des VER. Hieraus wird klar, daß eine Auswertung der Signalmorphologie für die AV-Intervallsteuerung vorteilhaft aufgrund eines Vergleiches der maximalen Signalamplituden im R--Bereich - für sich genommen - sowie der Relationen zwischen den Amplituden im R+- und im T+-Bereich möglich ist. Zur mathematischen Auswertung der letztgenannten Relation eignet sich besonders die Berechnung des Anstieges Δ0/Δt zwischen den lokalen Maxima R+ₘₐₓ und T+ₘₐₓ der einzelnen VER-Signalverläufe im Bereich der R+- und der T+-Welle. Die Anstiegsgeraden (ΔU/Δt)₁ und (ΔU/Δt)₂ sind in der Figur ebenfalls dargestellt.

Innerhalb einer vorgegebenen Menge von AV-Intervallen ist gemäß einem bevorzugten Optimierungsalgorithmus jener Intervallwert als optimal anzusehen, bei dem sowohl R-ₘₐₓ als auch ΔU/Δt minimal wird; es sind aber auch abweichende Optimierungs- bzw. Bewertungskriterien möglich.

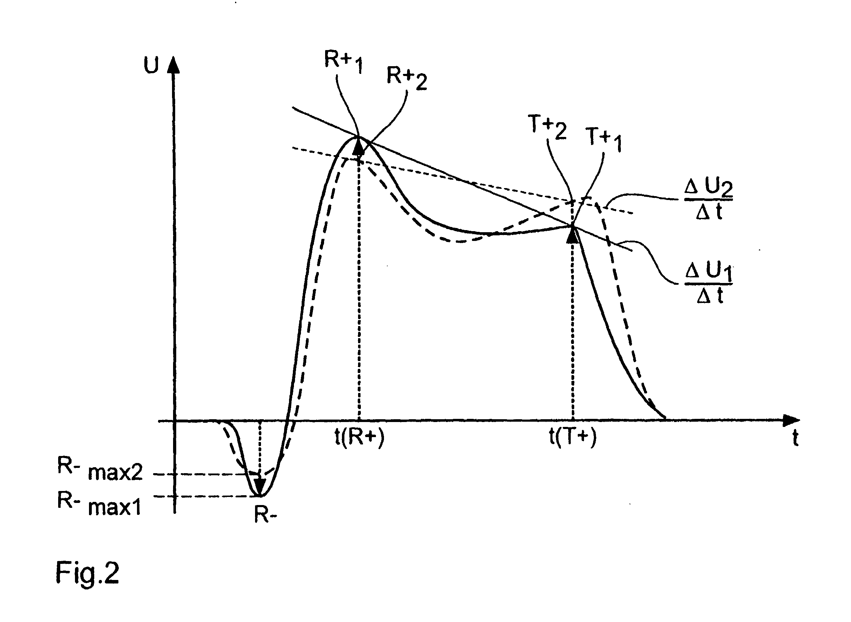

Fig. 2 ist eine zu Fig. 1 ähnliche Darstellung zweier verschiedener VER-Signalverläufe. Bezüglich der einzelnen Größen wird daher auf die obige Erläuterung zu Fig. 1 hingewiesen. Abweichend von Fig. 1, wird hier aber nicht die Relation der Maximalamplituden der R+-Welle und T+-Welle ausgewertet, sondern die Relation der Amplituden zu vorgegebenen Zeitpunkten t(R+) und t(T+). Es werden also die Steigungen ΔU₁/Δt bzw. ΔU₂/Δt zwischen den Punkten R+₁ und T+₁ bzw. R+₂ und T+₂ bestimmt und ausgewertet. Dies verein facht die Auswertung des Signalverlaufes erheblich und sit für bestimmte Optimierungsanwendungen hinreichend, obgleich die zeitliche Lage des lokalen Maximums im Bereich der T+-Welle nicht belastungsunabhängig ist.

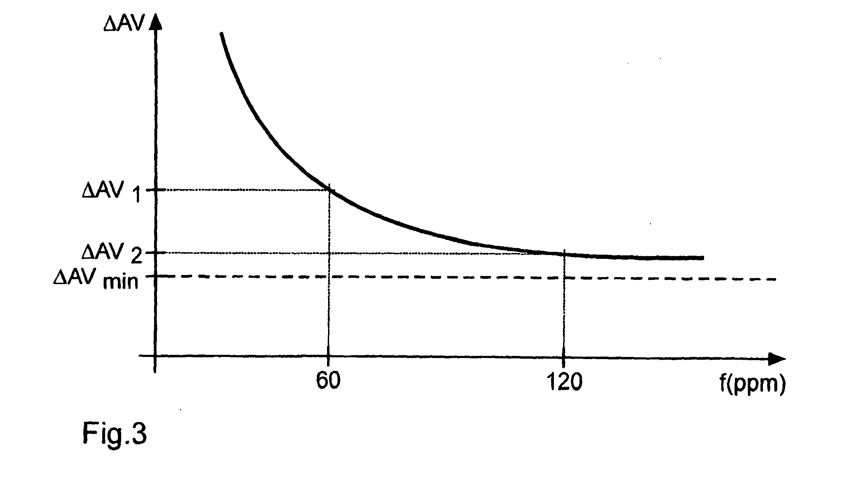

Fig. 3 gibt eine schematische Darstellung der Abhängigkeit des AV-Intervalls ΔAV von der Stimulationsfrequenz f, der sogenannten Bezeet-Kurve, die zur Einstellung des optimierten AV-Intervalls für beliebige Werte der Stimulationsfrequnez aufgrund zweier Kalibrierungswerte (in der Figur mit ΔAV₁ bzw. ΔAV₂ bezeichnet) benutzt wird.

Zur Kalibrierung wird bei mindestens zwei festen Stimulationsfrequenzen (im Beispiel 60 bzw. 120 ppm) jeweils ein AV-Intervall-Wertebereich überstrichen und die zugehörige Meßwertmenge der Signalparameter gemäß den obigen Ausführungen nach mindestens einem vorab definierten Kriterium bewertet, um das für die fixe Frequenz optimale AV-Intervall zu finden. Zwei Meßreihen bei einer niedrigen sowie einer hohen Frequenz und unverändertem Patientenzustand - bevorzugt im liegenden und ruhenden Zustand - reichen in der Regel aus, um die gültige Bezeet-Kurve zu finden und damit für jeden im nachfolgenden Schrittmacher-Normalbetrieb auftretenden Frequenzwert einen zugehörigen spezifischen AV-Intervallwert verfügbar zu haben.

Da sich die evozierten Herzsignale nur bei Stimulation ausbilden und intrinsische Signale sich nicht zur vorstehend skizzierten Bestimmung eines Signalformparameters heranziehen lassen, ist für die AV-Intervall-Anpassung für die Dauer des Kalibrationsvorganges eine phasenweise ununterbrochene P-synchrone ventrikuläre Stimulation auch bei u.U. adäquater Eigenfrequenz des Patienten zu gewährleisten. Darüber hinaus muß wegen der mit Fusion-Beats (nahezu gleichzeitiges Auftreten von Stimulationsimpuls und spontaner Herzaktion) verbundenen unphysiologischen Beeinflussung des Differenzparameters das Vorkommen von Fusion-Beats vermieden werden. Um neben der eher unproblematischen Meßreihe bei hoher Frequenz (Überstimulation) auch eine Meßreihe bei niedriger Stimulationsfrequenz aufnehmen zu können, sollte das Vorliegen eines Zustandes geringer intrinsischer Herzrate für die Dauer der Messungen gewährleistet sein. Falls dies durch Auswahl einer Ruhephase nicht hinreichend möglich sein sollte, können unterstützend Medikamentengaben erfolgen. Bis zu einem gewissen Grade kann auch eine Anhebung der unteren Meßfrequenz über die intrinsische Rate noch hinreichend genaue Messungen ermöglichen.

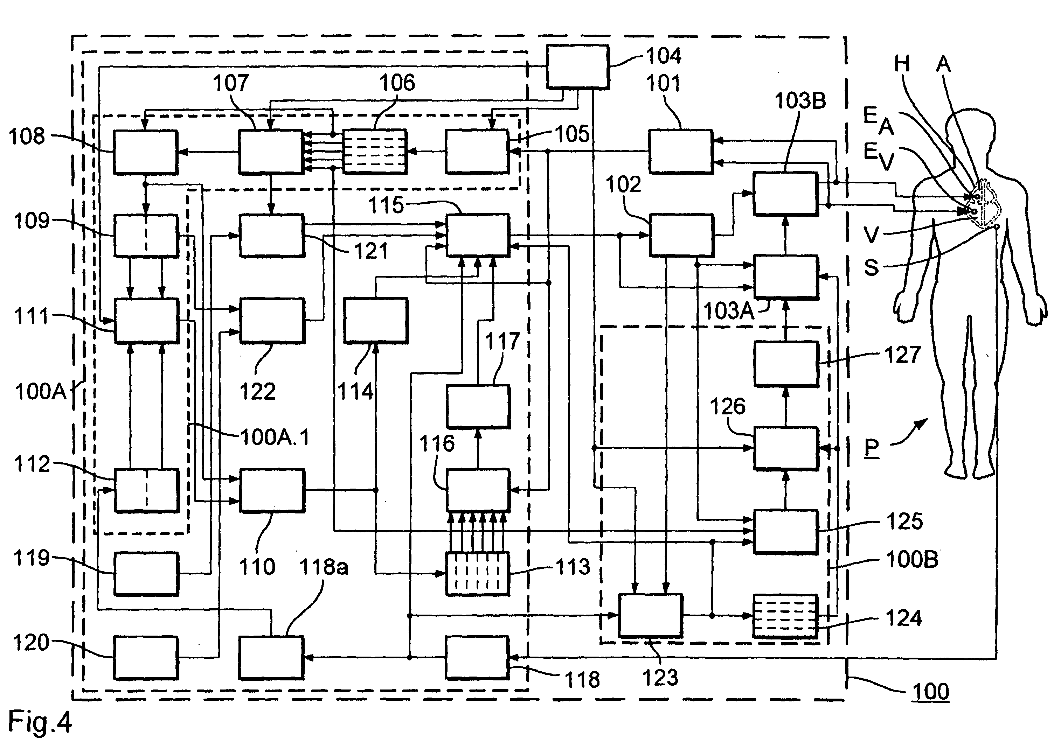

In Fig. 4 sind in Form eines Funktions-Blockschaltbildes wesentliche Elemente eines ratenadaptiven Herzschrittmachers 100 gezeigt, wobei im Interesse der Übersichtlichkeit an sich bekannte, für die Ausführung der Erfindung jedoch nicht wesentliche Schrittmacherkomponenten fortgelassen sind.

Der Herzschrittmacher 100 ist ein- und ausgangsseitig mit zwei intrakardialen Elektroden E_{A} im Atrium A und E_{V} im Ventrikel V des Herzens H eines Patienten P und einem kombinierten Positions- und Aktivitätssensor S verbunden. Über die Ventrikelelektrode E_{V} erfaßte ventrikuläre Herzsignale werden dem Eingang einer zur signalformgetreuen Erfassung intrakardialer EKG-Signale ausgebildeten (als solche bekannten) Herzsignal-Eingangsstufe 101 zugeführt. Auch an der atrialen Elektrode E_{A} auftretende Vorhofsignale werden der Herzsignal-Eingangsstufe 101 zugeführt; diese werden aber nur in üblicher Weise zur Festlegung des Stimulations-Timings herangezogen, ohne daß ihre Morphologie ausgewertet würde. Durch einen Stimulationsimpulsgenerator 102 erzeugte Stimulationsimpulse werden über eine AV-Verzögerungseinheit 103A und eine Ausgangsstufe 103B und die Elektroden E_{A} und E_{V} an das Atrium A bzw. den Ventrikel V abgegeben.

Der Schrittmacher 100 enthält eine Ablaufsteuerung 104, die den gesamten Betriebsablauf steuert - in der Figur sind aber im Interesse der Übersichtlichkeit nur die Steuersignalverbindungen zu einigen im gegebenen Zusammenhang wichtigen Komponenten gezeigt.

Die Erzeugung und Bereitstellung der Ratensteuersignale zur Steuerung der Stimulationsrate entsprechend dem physiologischen Bedarf des Patienten P erfolgt in einer eingangsseitig sowohl mit der Herzsignal-Eingangsstufe 101 als auch dem Aktivitätssensor S und ausgangsseitig mit dem Stimulationsimpulsgenerator 102 verbundenen Ratenberechnungseinrichtung 100A, die als wesentlichsten Funktionsblock eine Signalamplituden-Verarbeitungseinheit 100A.1 aufweist.

Die Signalamplituden-Verarbeitungseinheit 100A.1 umfaßt eine über eine (ihrerseits durch die Ablaufsteuerung 104 getriggerte) A/D-Wandler- und Speicherzugriffseinheit 105 mit der Herzsignal-Eingangsstufe 101 verbundene serielle Herzsignal-Speichereinrichtung 106 mit einer Mehrzahl von Speicherbereichen für jeweils eine vollständige evozierte ventrikuläre Herzaktion (VER). Mit dem Ausgang der Speichereinrichtung 106 sind eine - ebenfalls durch die Ablaufsteuerung 104 gesteuerte - Variations-Auswertungseinheit 107 und eine Subtraktionsstufe 108 verbunden.

In der Variations-Auswertungseinheit 107 werden für einen vorbestimmten Zeitraum oder eine vorbestimmte Anzahl von evozierten Herzsignalen (entsprechend einem in der Ablaufsteuerung 104 gespeicherten Programm einmalig oder in vorbestimmten größeren Abständen) diejenigen Abschnitte bzw. Zeitpunkte des VER-Signals ermittelt, für die die Differenz der Signalamplituden maximale Variabilität zeigt. In der Subtraktionsstufe 108 wird fortlaufend für das jeweils letzte aufgenommene Herzsignal U(t) der aktuelle Amplitudendifferenzwert ΔU = U₂(t₂) - U₁(t₁) zu diesen beiden Zyklus-Zeitpunkten (auch als Adaptions- oder Differenzparameter bezeichnet) gebildet. Ausgangsseitig ist die Subtraktionsstufe mit einem Amplitudendifferenzspeicher 109 sowie einer Umrechnungsstufe 110 verbunden. Im Amplitudendifferenzspeicher 109 werden - fortlaufend aktualisiert - der jeweils erfaßte größte und kleinste Amplitudendifferenzwert festgehalten und einem Eingangspaar einer Normierungsstufe 111 zugeführt, die über ein zweites Eingangspaar mit einem Ratengrenzwertspeicher 112 verbunden ist, in dem die für die Ratensteuerung minimal und maximal zulässige Stimulationsrate gespeichert sind.

In der Normierungsstufe 111 wird aus dem jeweils vorliegenden Amplitudendifferenz-Minimal- und -Maximalwert und dem Stimulationsraten-Minimal- und -Maximalwert der aktuell gültige physiologische Verlauf des Differenzparameters in Abhängigkeit von der Stimulationsrate ermittelt und der Umrechnungsstufe 110 zugeführt. In letzterer wird hieraus und aus dem aus der Subtraktionsstufe 108 erhaltenen aktuellen Wert des Differenzparameters gemäß dem oben erläuterten Vorgehen das aktuelle Ratensteuersignal errechnet.

Die Umrechnungsstufe 110 ist ausgangsseitig parallel mit einem Ratensteuersignalspeicher 113 sowie einer Glättungsstufe 114 verbunden. Die Glättungsstufe 114, in der (auf an sich bekannte Weise) eine Nachverarbeitung des Ratensteuersignals zur Vermeidung zu großer "Ratensprünge" erfolgt, ist mit einem Eingang einer Umschalteinheit 115 verbunden, über die das Ratensteuersignal unter den nachfolgend angegebenen Bedingungen letztlich zum Stimulationsimpulsgenerator 102 durchgeschaltet wird.

Der Ratensteuersignalspeicher 113 ist mit dem Eingang einer Trendberechnungsstufe 116 verbunden, die über eine Steuersignalverbindung mit der Herzsignal-Eingangsstufe 101 verbunden ist und über diese aktiviert wird, falls kein VER-Signal erfaßt wird. Die Trendberechnungsstufe 116 lädt nach ihrer Aktivierung die gespeicherten Ratensteuersignale in zeitlicher Zuordnung und führt hierfür eine Trendberechnung durch, deren Ergebnis an eine Ersatzsteuersignal-Berechnungseinheit 117 ausgegeben wird. Diese berechnet ihrerseits in Fortschreibung des errechneten Trends ein Ersatz-Ratensteuersignal. Dieses wird durch die - ebenfalls über die Herzsignal-Eingangsstufe 101 aktivierte - Umschalteinheit 115 zur Ersetzung des bei Fehlen eines VER-Signals nicht verfügbaren originären Ratensteuersignals zum Stimulationsimpulsgenerator durchgeschaltet.

Der Positions- und Aktivitätsfühler S ist mit einer Fühlersignal-Verarbeitungseinheit 118 verbunden, die - gemäß bekannten Algorithmen - aus dem Aktivitätssignal ein weiteres Ersatz-Ratensteuersignal berechnet, das an einem weiteren Eingang der Umschalteinheit 115 bereitgestellt wird. Zudem bildet die Fühlersignal-Verarbeitungseinheit 118 den jeweils gültigen Stimulationsraten-Maximalwert und übergibt diesen an den Speicher 112.

Die Ratenberechnungseinrichtung 100A umfaßt weiterhin einen Variationsbreitenspeicher 119 und einen Amplitudendifferenz-Maximalwertspeicher 120, in denen als für den Anwendungsbereich der vorgeschlagenen Lösung wesentliche Randbedingungen einerseits ein minimal zulässiger Amplitudenvaribilitätswert und andererseits der maximal zulässige Amplitudendifferenz- bzw. Differenzparameterwert gespeichert sind. Die gespeicherten Werte werden in einer Variabilitäts-Vergleichereinheit 121 bzw. einer Amplitudendifferenz-Vergleichereinheit 122 einem Vergleich mit den jeweiligen Ist-Werten unterzogen, die den Vergleichereinheiten 121, 122 von der Variations-Auswertungseinheit 107 bzw. vom Amplitudendifferenzspeicher 109 zugeführt werden. Ergibt der Vergleich, daß eine der geforderten Randbedingungen nicht erfüllt ist, wird durch Ausgabe eines entsprechenden Steuersignals durch die Vergleichereinheiten 121, 122 an die Umschalteinheit 115 anstelle des aufgrund der Signalmorphologie errechneten Ratensteuersignals das durch die Fühlersignal-Verarbeitungseinheit 118 erzeugte Ersatz-Ratensteuersignal an den Stimulationsimpulsgenerator durchgeschaltet.

Neben der Ratenberechnungseinrichtung 100A weist der Schrittmacher 100 eine AV-Intervall-Berechnungseinrichtung 100B auf, die ebenfalls über die Ablaufsteuerung 104 aktiviert und gesteuert wird. Die Aktivierung erfolgt über ein zweistufiges UND-Gatter 123, dessen erser Eingang mit dem Ausgang der Ablaufsteuerung 104, dessen zweiter Eingang mit dem Ausgangs des Stimulationsimpulsgenerators und dessen dritter Eingang mit dem Ausgang der Fühlersignal-Verarbeitungseinheit 118 verbunden ist. Das UND-Gatter 123 läßt ein (periodisch ausgegebenes) Aktivierungssignal der Ablaufsteuerung 104 zur Einleitung einer AV-Intervallbestimmung nur dann passieren, wenn zugleich ein Ausgangssignal der Fühlersignal-Verarbeitungseinheit 118, das einen liegenden bzw. Ruhezustand des Patienten P anzeigt, und ein die Ausgabe von Stimulationsimpulsen kennzeichendes Signal vom Stimulationsimpulsgenerator anliegen. Dadurch wird gesichert, daß ein AV-Einstellzyklus nur ausgeführt wird, wenn der Patient sich in Ruhe bzw. im liegenden Zustand befindet und der Schrittmacher nicht inhibiert ist, d.h. die erfaßten Herzsignale tatsächlich evozierte Potentiale repräsentieren.

Sind diese Bedingungen erfüllt, wird über das UND-Gatter 123 für eine vorbestimmte Meßreihe einerseits an der Umschalteinheit 115 eine erste vorbestimmte Stimulationsrate fest eingestellt und andererseits aus einem AV-Intervall-Vorwahlspeicher 124 sequentiell eine Folge von AV-Intervallwerten an die AV-Verzögerungseinheit 103A übergeben. Mit jedem dieser Werte wird eine - in der Ablaufsteuereinheit 104 programmierte - Anzahl von Zweikammerstimulationen ausgeführt und jeweils in den Stufen 101 und 105 eine stabilisierte. digitalisierte VER-Signalform gewonnen und im Speicher 106 abgespeichert.

Während oder nach der ersten Meßreihe bei der ersten Stimulationsfrequenz wird in einer Signalform-Auswertungseinheit 125 eine oben prinzipiell skizzierte und weiter unten genauer beschriebene Auswertung jeder einzelnen der im Speicher 106 zwischengespeicherten VER-Signalformen vorgenommen, In einer nachgeschalteten Meßreihen-Bewertungseinheit 126, der aus dem Vorwahlspeicher 124 der zu jeder Signalformkurve korrespondieredne AV-Intervallwert zugeführt wird, wird eine zusammenfassende Bewertung der Auswertungsergebnisse der Meßreihe anhand mindestens eines intern gespeicherten Bewertungskriteriums durchgeführt. Am Ausgang der Meßreihen-Bewertungseinheit 126 steht dann der zur ersten eingestellten Stimulationsfrequenz korrespondierende optimale AV-Intervallwert bereit und wird einer Bezeetkurven-Auswahleinheit 127 zugeführt.

Sobald dies geschehen ist, wird über die Ablaufsteuereinheit 104 eine zweite, gleichartige Meßreihe bei einer zweiten fest eingestellten Stimulationsfrequenz initiiert und ausgeführt, in deren Ergebnis ein zur zweiten Stimlationsfrequenz korrespondierendes optimales AV-Intervall ermittelt und an die Bezeetkurven-Auswahleinheit 127 übergeben wird. Nach Empfang läuft dort anhnd der beiden vorliegenden Werte ΔAV1(f1) und ΔAV2(f2) eine Auswahlprozedur aus einer Schar vorgespeicherter Funktionskurven ab, in deren Ergebnis die aktuell gültige Funktionskurve ΔAV = f(f) vorliegt.

Diese wird an die AV-Verzögerungseinheit 103A übergeben und dort gespeichert. Die AV-Verzögerungseinheit ist neben dem Stimulationsimpulsgenerator 102 direkt mit dem Ausgang der Stimulationsfrequenz-Umschalteinheit 115 verbunden und ordnet aufgrund der gespeicherten Bezeet-Kurve im weiteren Betrieb des Schrittmachers jedem von dort überittelten aktuellen Wert der Stimulationsfrequenz ein spezifisches AV-Intervall zu, um das der nächste abzugebende Kammerstimulus gegenüber dem zugehörigen Vorhofstimulus verzögert wird.

In dem Fall, daß turnusmäßige Kalibrationszyklen aufgrund einer dauerhaften Inhibierung des Schrittmachers oder aus anderen Gründen nicht ausgeführt werden können, arbeitet die AV-Verzögerungseinheit 103A mit einem oder mehreren programmierten, jeweils fest einer Stimulationsfrequenz zugeordneten AV-Intervall-Ersatzwert (en). Alternativ kann auch die AV-Intervall-Berechnungseinheit 100B - analog wie weiter oben für die Ratenberechnungseinheit 100A beschrieben - Mittel zur Extrapolation von Vergangenheitswerten umfassen, die eine Fortschreibung eines beobachteten Trends ermöglichen.

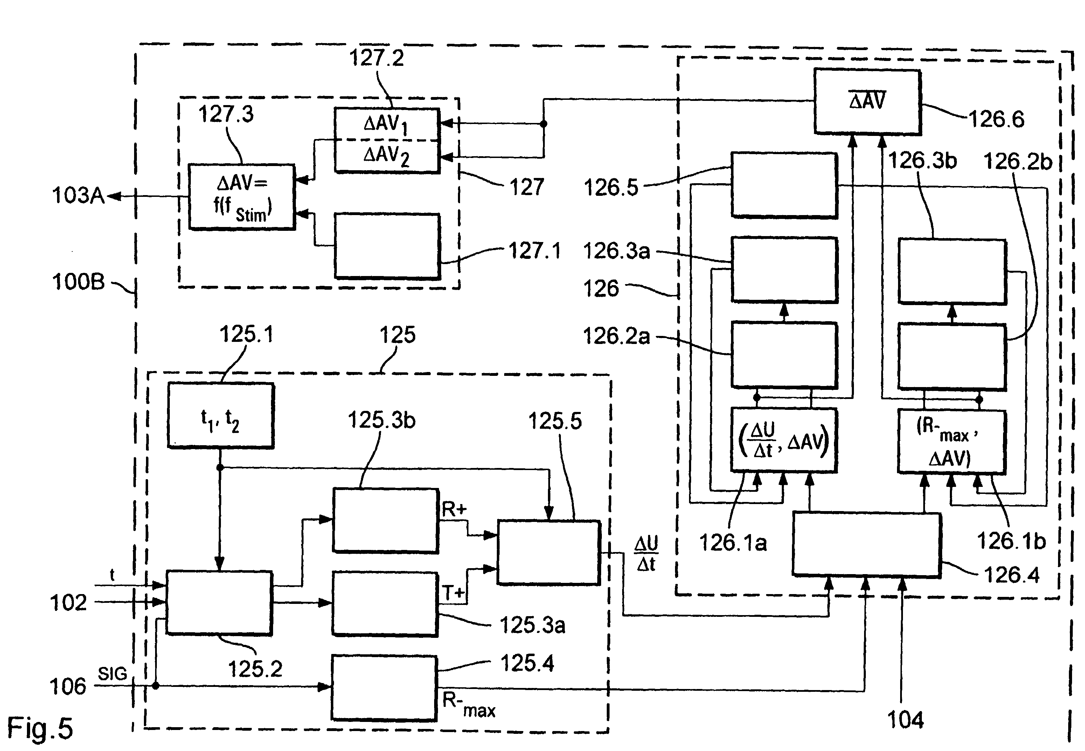

Fig. 5 zeigt - ebenfalls in Form eines Funktions-Blockschaltbildes - Aufbau und Funktion der wichtigsten Komponenten der AV-Intervall-Berechungseinheit 100B aus Fig. 4 genauer.

Die Signalform-Auswertungseinheit 125 weist in der in Fig. 4 und 5 gezeigten Ausführung eingangsseitige Verbindungen sowohl zum Stimulationsimpulsgenerator 102 als auch zur Herzsignal-Speichereinrichtung 106 und zudem natürlich (was in Fig. 4 nicht dargestellt und in Fig. 5 durch ein Eingangssignal "t" symbolisiert ist) zu einem zentralen Zeitgeber des Schrittmachers auf. Sie empfängt somit neben der eigentlichen Signalforminformation SIG Zeitinformationen bezüglich des Stimulusimpulses sowie des durch den Stimulusimpuls evozierten Herzsignals. Die Zuammenführung der einzelnen Zeitinformationen zu zeitlich auf den jeweiligen Stimulusimpuls bezogenen Herzsignalverläufen und zugleich die Zuordnung zweier in einem internen Arbeitsspeicher 125.1 gespeicherter Abtastzeitpunkte t(R+), t(T+) (vgl. Fig. 2) erfolgt in einer Abtastzeit-Korrelationsstufe 125.2. Deren Ausgang ist mit zwei Amplitudendetektoren 125.3a und 125.3b verbunden. Diese erfassen die Amplitudenwerte des Herzsignals zu den Zeitpunkten t(R+) bzw. t(T+), während ein parallelgeschalteter mehrstufiger Amplitudendiskriminator 125.4 die Maximalamplitude im Bereich negativer Signalpolarität, d.h. die Amplitude R-ₘₐₓ der jeweiligen R--Welle, erfaßt.

Die Amplitudendetektoren 125.3a, 125.3b sind ausgangsseitig mit einer arithmetischen Berechnungseinheit 125.5 verbunden, die zudem aus dem Arbeitsspeicher 125.1 die Abtastzeitpunkte erhält und aus den abgetasteten Amplitudenwerten und Abtastzeitpunkten die Steigung ΔU/Δt des Herzsignals im Bereich von der R+- zur T+-Welle (Fig. 2) berechnet. Am Ausgang der Signalform-Auswertungseinheit 125 stehen somit im Ergebnis der Auswertung jedes einzelnen Herzsignals jeweils die Signalformparameter R-ₘₐₓ und ΔU/Δt bereit.

Die Meßreihen-Bewertungseinheit 126 enthält je einen Zweibereichs-Parameterspeicher 126.1a, 126.1b zur Speicherung der Signalformparameterpaare jeweils einer früheren sowie der letzten analysierten Herzsignalkurve zusammen mit dem korrespondierenden AV-Intervallwert und je eine den Paramterspeichern 126.1a, 126.1b zugeordnete Vergleichereinheit 126.2a, 126.2b. Die Eingänge der Vergleichereinheiten sind jeweils mit den beiden Speicherbereichen verbunden, in denen die Parameterwerte des aktuellen und des früheren Herzsignals gespeichert sind, und diese werden miteinander verglichen. Durch ein im Ergebnis des Vergleiches ausgegebenes Steuersignal wird jeweils eine Speicherlöschsteuerung 126.3a bzw. 126.3b aktiviert, die den Inhalt des Speicherbereiches mit dem jeweils größeren Wert R-ₘₐₓ bzw. ΔU/Δt löscht. Im Ergebnis der sequentiellen Morphologievergleiche bleibt mithin Schritt für Schritt stets der bis dahin kleinste Wert des Signalformparameters (zusammen mit dem zugehörigen AV-Intervallwert) gespeichert und wird zum Vergleich mit dem aktuell analysierten Herzsignal herangezogen. Dessen Parameterwerte werden dazu durch eine eingangsseitig mit der Ablaufsteuereinheit 104 verbundene Speicherschreibsteuerung 126.4 in die durch den Löschvorgang freigewordenen Speicherbereiche der Speicher 126.1a, 126.1b eingeschrieben.

Nach Abschluß jeder Meßreihe bei einer festen Stimulationsfrequenz sind in den Speichern 126.1a, 126.1b die Minimalwerte (R-ₘₐₓ)ₘᵢₙ bzw. (ΔU/Δt)ₘᵢₙ und der jeweils zugehörige AV-Intervallwert verblieben. Die beiden AV-Intervallwerte werden durch eine Speicherzugriffssteuerung 126.5 an eine Mittelungsstufe 126.6 übergeben, wo der aus der Auswertung beider Signalformparameter gemittelte AV-Intervallwert berechnet wird. In der Mittelungsstufe 126.6 kann eine arithmetische Mittelwertbildung aus dem zu (R-ₘₐₓ)ₘᵢₙ und dem (ΔU/Δt)ₘᵢₙ gehörigen AV-Intervallwert implementiert sein. Der gespeicherte Algorithmus kann aber auch eine Höhergewichtung bzw. Priorisierung eines der Signalformparameter für die Bestimmung des als insgesamt optimal anzusehenden AV-Intervalls einschließen.

Die Bezeetkurven-Auswahleinheit 127 umfaßt einen Funktionskurvenspeicher 127.1, in dem eine Schar von Funktionskurven ΔAV = f (f_{Stim}) vorgespeichert ist, und einen mit der Mittelungsstufe 126.6 verbundenen Zweibereichs-AV-Speicher 127.2, in dessen beide Speicherbereiche im Zuge einer Kalibrationsprozedur die für zwei Stimulationsfrequenzen als optimal ermittelten AV-Intervallwerte eingeschrieben werden. Kern der Auswahleinheit 127 ist eine Anpaßstufe 127.3 mit üblichem Aufbau, in der nach Empfang des zweiten AV-Intervallwertes der Kalibration von der Mittelungsstufe 126.6 selbsttätig aufgrund der im Speicher 127.2 abgelegten AV-Intervallwerte die gültige Bezeetkurve (Fig. 3) ermittelt wird.

Die Erfindung beschränkt sich in ihrer Ausführung nicht auf die oben erläuterten bevorzugten Ausführungsbeispiele. Vielmehr ist eine Anzahl von Varianten denkbar, welche von der dargestellten Lösung auch bei anders gearteten Ausführungen Gebrauch machen.

So ist neben der Ausführung in Gestalt des oben skizzierten Schrittmachers mit automatischer Anpassung des AV-Intervalls auch die Ausführung mittels einer Schrittmacher-Programmer-PC-Konfiguration im Rahmen von Nachsorgeuntersuchungen möglich. Hierbei kann ein optimiertes AV-Intervall für einen herkömmlichen programmierbaren Schrittmacher mittels eines auf dem externen Rechner implementierten AV-Intervall-Berechnungsalgorithmus ermittelt und anschließend in üblicher Weise mittels des Programmiergerates im Schrittmacher programmiert werden. Handelt es sich speziell um einen Schrittmacher, bei dem eine Zuordnung zwischen verschiedenen AV-Intervall- und Stimulationsratenwerten vorgesehen ist, ist auf diese Weise - dem oben skizzierten Prinzip folgend - die gespeicherte Zuordnungstabelle aktualisierbar.

Neben einem ratenadaptiven Zweikammerschrittmacher kann die Erfindung grundsätzlich auch in anderen Herzstimulationsgeräten realisiert sein, bei denen die Einhaltung eines optimierten Zeitabstandes zwischen einem atriellen Ereignis und einem ventrikulären Stimulus bedeutsam ist, etwa bei atriumsynchron arbeitenden Einkammerschrittmachern und bestimmten Defibrillatoren bzw. Kardiovertern.

## Patentansprüche

1. Herzstimulatoranordnung (100) mit
einem Stimulationsimpulsgenerator (102) und mit diesem verbundenen Ausgangsmitteln (103B, EV) zur Abgabe von Stimutationsimpulsen an den Ventrikel (V) eines Herzens (H),
einer Herzsignal-Eingangsstufe (101) zur Erfassung evozierter Herzsignale, insbesondere der ventrikulären evozierten Reizantwort VER,
einer ausgangsseitig mit dem Stimulationsimpulsgenerator verbundenen AV-Verzögerungseinheit (103A) zur Erzeugung eines AV-Intervalls (DAV) zwischen einem atriellen Herzereignis oder atriellen Stimulationsimpuls und einem an den Ventrikel abgegebenen Stimulationsimpuls,
einer eingangsseitig mit der Herzsignal-Eingangsstufe und ausgangsseitig mit der AV-Verzögerungseinheit verbundenen AV-Intervall-Berechnungseinrichtung (100B) zur Berechnung des AV-Intervalls aufgrund mindestens eines Parameters des Herzsignals,
**dadurch gekennzeichnet, dass**
die Herzsignal-Eingangsstufe zur Signalformerfassung evozierter Herzsignale, nämlich der Morphologie der ventrikulären evozierten Reizantwort VER, ausgebildet ist und dass Mittel (125.4) zur Erfassung der Maximalamplitude (R-max) der R- -Welle und/oder der zeitlichen Lage und Amplitude der Maxima der R+ -Welle und der T+ - Welle der VER vorgesehen sind, und
die AV-Intervall-Berechnungseinrichtung zur Berechnung des AV-Intervalls aufgrund der Maximalamplitude der R- -Welle und/oder der zeitlichen Lage und Amplitude der Maxima der R+ -Welle und der T+ - Welle ausgebildet ist.

2. Herzstimulatoranordnung (100) mit
einem Stimulationsimpulsgenerator (102) und mit diesem verbundenen Ausgangsmitteln (103B, EV) zur Abgabe von Stimulationsimpulsen an den Ventrikel (V) eines Herzens (H),
einer Herzsignal-Eingangsstufe (101) zur Erfassung evozierter Herzsignale, insbesondere der ventrikulären evozierten Reizantwort VER,
einer ausgangsseitig mit dem Stimulationsimpulsgenerator verbundenen AV-Verzögerungseinheit (103A) zur Erzeugung eines AV-Intervalls (DAV) zwischen einem atriellen Herzereignis oder atriellen Stimulationsimpuls und einem an den Ventrikel abgegebenen Stimulationsimpuls,
einer eingangsseitig mit der Herzsignal-Eingangsstufe und ausgangsseitig mit der AV-Verzögerungseinheit verbundenen AV-Intervall-Berechnungseinrichtung (100B) zur Berechnung des AV-Intervalls aufgrund mindestens eines Parameters des Herzsignals,
**dadurch gekennzeichnet,**
**dass** die Herzsignal-Eingangsstufe zur Signalformerfassung evozierter Herzsignale, nämlich der Morphologie der ventrikulären evozierten Reizantwort VER, ausgebildet ist und dass Mittel (125.4) zur Erfassung der Maximalamplitude (R-max) der R- -Welle und/oder ein Zeitgeber (t) zur Vorgabe je eines Amplitudenwert-Erfassungszeitpunktes (t1, t2) und Mittel (125.3a, 125,2b) zur Erfassung der Amplitudenwerte (R+, T+) zum jeweiligen Erfassungszeitpunkt im Bereich der R+ - Welle und der T+ -Welle der VER vorgesehen sind
und die AV-Intervall-Berechungseinrichtung (100B) zur Berechnung des AV-Intervalls aufgrund der Maximalamplitude der R- -Welle und/oder der Amplitudenwerte im Bereich der R+ -Welle und der T+ - Welle ausgebildet ist.

3. Herzstimulatoranordnung nach Anspruch 2, **dadurch gekennzeichnet, dass** die Erfassungszeitpunkte (t1, t2) invariant bei der Variation des AV-Intervalls und/oder gegenüber Änderungen der Stimulationsrate gehalten werden.

4. Herzstimulatoranordnung nach einem der Ansprüche 1 bis 3, **dadurch gekennzeichnet, dass** die AV-Intervall-Berechnungseinrichtung (100B) arithmetische Mittel (125.5) zur Bestimmung der Steigung (ΔU/Δt) der VER zwischen den Maxima bzw. den Amplitudenwert-Erfassungszeitpunkt-Wertepaaren der R+ -Welle und der T+ -Welle aufweist.

5. Herzstimulatoranordnung nach einem der vorangehenden Ansprüche, **gekennzeichnet durch**
einen AV-Intervall-Vorwahlspeicher (124) zur Speicherung einer vorbestimmten Menge von AV-Intervallwerten,
eine Ablaufsteuereinheit (105, 123) zur Ablaufsteuerung eines AV-Intervall-Einstellzyklus unter sukzessiver, temporärer Einstellung mehrerer gespeicherter AV-Intervallwerte,
eine Herzsignal-Speichereinrichtung (106; 125.2) zur Speicherung einer Mehrzahl von während des AV-Einstellzyklus erfassten Herzsignalverläufen in Zuordnung zum jeweils eingestellten AV-Intervallwert,
eine mit der Herzsignal-Speichereinrichtung verbundene Signalform-Auswertungseinheit (125, 126), die aus jedem gespeicherten Herzsignalverlauf mindestens einen Signalformparameterwert ermittelt und die ermittelten Signalformparameterwerte einer Auswertung anhand eines programmierten Bewertungskriteriums unterzieht, und
eine AV-Intervall-Auswahleinheit (127) zur Einstellung desjenigen AV-Intervallwertes oder derjenigen funktionalen Abhängigkeit zwischen AV-Intervallwerten und Stimulationsfrequenzwerten, der/die dem **durch** die Signalform-Auswertungseinheit als optimal bewerteten Herzsignalverlauf zugeordnet ist.

6. Herzstimulatoranordnung nach Anspruch 5, in Kombination mit Anspruch 1 oder in Kombination mit den Ansprüchen 1 und 4, **dadurch gekennzeichnet, dass** das programmierte Bewertungskriterium den Amplitudenwert der R- -Welle und/oder den Betrag der Steigung der VER zwischen den Maxima der R+ -Welle und der T+ -Welle betrifft.

7. Herzstimulatoranordnung nach einem der vorangehenden Ansprüche, **dadurch gekennzeichnet, dass** die AV-Intervall-Berechnungseinrichtung (100B) oder die AV-Verzögerungseinheit (103A) zur Ausgabe eines AV-Intervall-Ersatzwertes bei Nichtvorliegen eines aktuellen AV-Intervall-Berechnungsergebnisses, insbesondere bei Inhibierung des Stimulationsimpulsgenerators (102) bzw. Nichterfassung eines evozierten Herzsignals, ausgebildet ist.

8. Herzstimulatoranordnung nach einem der vorangehenden Ansprüche, **gekennzeichnet durch** einen Positions- und/oder Aktivitätsfühler (S) und eine über einen Steuereingang mit diesem verbundene Ablaufsteuerung (105, 123) zur Steuerung einer AV-Intervall-Kalibrierungsprozedur in Abhängigkeit vom Ausgangssignal des Positions- und/oder Aktivitätsfühlers.

9. Herzstimulatoranordnung nach einem der vorangehenden Ansprüche, **gekennzeichnet durch** die Ausführung als raten-adaptiver Herzschrittmacher (100) mit Mitteln (100A) zur belastungsabhängigen Einstellung der Stimulationsfrequenz und Mitteln (127) zur Zuordnung eines spezifischen AV-Intervallwertes zu mindestens einer Teilmenge der Stimulationsfrequenzwerte.

10. Herzstimulatoranordnung nach einem der vorangehenden Ansprüche, **gekennzeichnet durch** die Ausführung als Zweikammerschrittmacher (100) mit Mitteln (103B, EA) zur Abgabe von Stimulationsimpulsen an das Atrium (A).

11. Herzstimulatoranordnung nach einem der vorhergehenden Ansprüche, **dadurch gekennzeichnet, dass** die AV-Intervall-Berechnungseinrichtung außerhalb eines implantierbaren Herzstimulators, insbesondere in einem Programmiergerät oder einem Zusatzgerät zu diesem, vorgesehen ist.

12. Herzstimulatoranordnung nach einem der Ansprüche 1 bis 10, **dadurch gekennzeichnet, dass** die AV-Intervall-Berechnungseinrichtung (100B) als Bestandteil eines implantierbaren Herzstimulators (100) ausgebildet ist.

## Claims

1. A cardiac stimulator arrangement (100) comprising
a stimulation pulse generator (102) and output means (103B, EV) connected thereto for the delivery of stimulation pulses to the ventricle (V) of a heart (H),
a cardiac signal input stage (101) for detecting evoked cardiac signals, in particular the ventricular evoked stimulus response VER,
an AV delay unit (103A) connected on the output side to the stimulation pulse generator for producing an AV interval (DAV) between an atrial cardiac event or atrial stimulation pulse and a stimulation pulse delivered to the ventricle,
an AV interval calculating device (100B) connected on the input side to the cardiac signal input stage and on the output side to the AV delay unit for calculating the AV interval on the basis of at least one parameter of the cardiac signal,
**characterised in that**
the cardiac signal input stage is adapted for signal shape detection of evoked cardiac signals, namely the morphology of the ventricular evoked stimulus response VER, and that there are provided means (125.4) for detecting the maximum amplitude (R-max) of the R- -wave and/or the time position and amplitude of the maxima of the R+ -wave and the T+ -wave of the VER, and
the AV interval calculating device is adapted for calculating the AV interval on the basis of the maximum amplitude of the R- -wave and/or the time position and amplitude of the maxima of the R+ -wave and the T+ - wave.

2. A cardiac stimulator arrangement (100) comprising
a stimulation pulse generator (102) and output means (103B, EV) connected thereto for the delivery of stimulation pulses to the ventricle (V) of a heart (H),
a cardiac signal input stage (101) for detecting evoked cardiac signals, in particular the ventricular evoked stimulus response VER,
an AV delay unit (103A) connected on the output side to the stimulation pulse generator for producing an AV interval (DAV) between an atrial cardiac event or atrial stimulation pulse and a stimulation pulse delivered to the ventricle,
an AV interval calculating device (100B) connected on the input side to the cardiac signal input stage and on the output side to the AV delay unit for calculating the AV interval on the basis of at least one parameter of the cardiac signal,
**characterised in that**
the cardiac signal input stage is adapted for signal shape detection of evoked cardiac signals, namely the morphology of the ventricular evoked stimulus response VER, and that there are provided means (125.4) for detecting the maximum amplitude (R-max) of the R- -wave and/or a timer (t) for specifying a respective amplitude value detection time (t1, t2) and means (125.3a, 125.2b) for detecting the amplitude values (R+, T+) at the respective detection time in the region of the R+ -wave and the T+ -wave of the VER, and
the AV interval calculating device (100B) is adapted for calculating the AV interval on the basis of the maximum amplitude of the R- -wave and/or the amplitude values in the region of the R+ -wave and the T+ - wave.

3. A cardiac stimulator arrangement according to claim 2 **characterised in that** the detection times (t1, t2) are kept invariant upon the variation in the AV interval and/or in relation to changes in the stimulation rate.

4. A cardiac stimulator arrangement according to one of claims 1 to 3 **characterised in that** the AV interval calculating device (100B) has arithmetic means (125.5) for determining the gradient (DU/Dt) of the VER between the maxima or the amplitude value detection time value pairs of the R+ -wave and the T+ -wave.

5. A cardiac stimulator arrangement according to one of the preceding claims **characterised by** an AV interval preselection memory (124) for storage of a predetermined amount of AV interval values,
a procedural control unit (105, 123) for procedural control of an AV interval setting cycle with successive temporary setting of a plurality of stored AV interval values,
a cardiac signal memory device (106; 125.2) for storing a plurality of cardiac signal patterns detected during the AV setting cycle in association with the respective set AV interval value,
a signal shape evaluation unit (125, 126) which is connected to the cardiac signal memory device and which ascertains at least one signal shape parameter value from each stored cardiac signal pattern and subjects the ascertained signal shape parameter values to evaluation on the basis of a programmed assessment criterion, and
an AV interval selector unit (127) for setting that AV interval value or that functional dependency between AV interval values and stimulation frequency values, that is associated with the cardiac signal pattern assessed as optimum by the signal shape evaluation unit.

6. A cardiac stimulator arrangement according to claim 5 in combination with claim 1 or in combination with claims 1 and 4, **characterised in that** the programmed assessment criterion concerns the amplitude value of the R- -wave and/or the magnitude of the gradient of the VER between the maxima of the R+ -wave and the T+ -wave.

7. A cardiac stimulator arrangement according to one of the preceding claims **characterised in that** the AV interval calculating device (100B) or the AV delay unit (103A) is adapted to output an AV interval substitute value in the absence of a current AV interval calculation result, in particular upon inhibition of the stimulation pulse generator (102) or nondetection of an evoked cardiac signal.

8. A cardiac stimulator arrangement according to one of the preceding claims **characterised by** a position and/or activity sensor (S) and a procedural control means (105, 123) connected thereto by way of a control input for controlling an AV interval calibration procedure in dependence on the output signal from the position and/or activity sensor.

9. A cardiac stimulator arrangement according to one of the preceding claims **characterised in that** it is in the form of a rate-adaptive cardiac pacemaker (100) with means (100A) for exertion-dependent setting of the stimulation frequency and means (127) for associating a specific AV interval value with at least a portion of the stimulation frequency values.

10. A cardiac stimulator arrangement according to one of the preceding claims **characterised in that** it is in the form of a dual-chamber cardiac pacemaker (100) with means (103B, EA) for the delivery of stimulation pulses to the atrium (A).

11. A cardiac stimulator arrangement according to one of the preceding claims **characterised in that** the AV interval calculating device is provided outside an implantable cardiac stimulator, in particular in a programming device or an accessory device.

12. A cardiac stimulator arrangement according to one of claims 1 to 10 **characterised in that** the AV interval calculating device (100B) is in the form of a component part of an implantable cardiac stimulator (100).

## Revendications

1. Dispositif de stimulateur cardiaque (100) comportant
un générateur d'impulsions de stimulation (102) et des moyens de sortie (103B, EV) reliés à ce dernier et servant à délivrer des impulsions de stimulation au ventricule (V) d'un coeur (H),
un étage (101) d'entrée de signaux cardiaques pour détecter des signaux cardiaques provoqués, notamment la réponse d'excitation ventriculaire provoquée VER,
une unité de retardement AV (103A), qui est reliée côté sortie au générateur d'impulsions de stimulation et sert à produire un intervalle AV (DAV) entre un événement cardiaque atrial ou une impulsion de stimulation atriale et une impulsion de stimulation délivrée au ventricule,
un dispositif (100B) de calcul de l'intervalle AV, relié côté entrée à l'étage d'entrée des signaux cardiaques et côté sortie à l'unité de retardement AV, pour le calcul de l'intervalle AV sur la base d'au moins un paramètre du signal cardiaque,
**caractérisé en ce que**
l'étage d'entrée de signaux cardiaques est conçu pour la détection de la forme de signaux cardiaques provoqués, à savoir la morphologie de la réponse d'excitation ventriculaire provoquée VER, et des moyens (125.4) sont prévus pour détecter l'amplitude maximale (R-max) de l'onde R- et/ou une position dans le temps et l'amplitude des maxima de l'onde R+ et de l'onde T+ de la réponse VER, et
le dispositif de calcul de l'intervalle AV est conçu pour calculer l'intervalle AV sur la base de l'amplitude maximale de l'onde R- et/ou de la position temporelle et de l'amplitude des maxima de l'onde R+ et de l'onde T+.

2. Dispositif de stimulateur cardiaque (100) comportant
un générateur d'impulsions de stimulation (102) et des moyens de sortie (103B, EV) reliés à ce dernier et servant à délivrer des impulsions de stimulation au ventricule (V) d'un coeur (H),
un étage (101) d'entrée de signaux cardiaques pour détecter des signaux cardiaques provoqués, notamment la réponse d'excitation ventriculaire provoquée VER,
une unité de retardement AV (103A), qui est reliée côté sortie au générateur d'impulsions de stimulation et sert à produire un intervalle AV (DAV) entre un événement cardiaque atrial ou une impulsion de stimulation atriale et une impulsion de stimulation délivrée au ventricule,
un dispositif (100B) de calcul de l'intervalle AV, relié côté entrée à l'étage d'entrée des signaux cardiaques et côté sortie à l'unité de retardement AV, pour le calcul de l'intervalle AV sur la base d'au moins un paramètre du signal cardiaque,
**caractérisé en ce**
**que** l'étage d'entrée de signaux cardiaques est agencé pour la détection de la forme de signaux cardiaques provoqués, à savoir la morphologie de la réponse impulsionnelle ventriculaire provoquée (VER) et qu'il est prévu des moyens (125.4) pour détecter l'amplitude maximale (R-max) de l'onde R- et/ou un générateur de temps (t) pour prédéterminer respectivement un instant (t1, t2) de détection de la valeur d'amplitude, et des moyens (125.3a, 125.2b) pour détecter les valeurs d'amplitude (R+, T+) à l'instant respectif de détection dans la région de l'onde R+ et de l'onde T+,
et **que** le dispositif de calcul de l'intervalles AV est conçu pour calculer l'intervalle AV sur la base de l'amplitude maximale de l'onde R- et/ou des valeurs d'amplitude de l'onde R+ et de l'onde T+.

3. Dispositif de stimulateur cardiaque selon la revendication 2, **caractérisé en ce que** les instants de détection (t1, t2) sont maintenus invariants lors de la variation de l'intervalle AV et/ou vis-à-vis de variations de la cadence de stimulation.

4. Dispositif de stimulateur cardiaque selon l'une des revendications 1 à 3, **caractérisé en ce que** le dispositif (100B) de calcul de l'intervalle AV comporte des moyens arithmétiques (125.5) pour déterminer la pente (DU/Dt) de la réponse VER entre les maxima ou les couples de valeurs valeur d'amplitude - instant de détection de l'onde R+ et de l'onde T+.

5. Dispositif de stimulateur cardiaque selon l'une des revendications précédentes, **caractérisé par**
une mémoire (124) de présélection d'intervalles AV pour la mémorisation d'une quantité prédéterminée de valeurs d'intervalles AV,
une unité de commande de déroulement (105, 123) pour commander le déroulement d'un cycle de réglage d'intervalles AV moyennant le réglage temporaire successif de plusieurs valeurs mémorisées d'intervalles AV,
un dispositif (106; 125.2) de mémorisation de signaux cardiaques pour mémoriser une multiplicité d'allures de signaux cardiaques détectées pendant le cycle de réglage AV, en association avec respectivement une valeur réglée d'intervalle de mesure,
une unité (125, 126) d'évaluation de la forme des signaux, qui est reliée au dispositif de mémoire de signaux cardiaques et détermine au moins une valeur de paramètre de forme de signal à partir de l'allure mémorisée du signal cardiaque et soumet les valeurs déterminées de paramètres de formes de signaux à une évaluation sur la base d'un critère programmé de pondération, et
une unité (127) de sélection d'intervalle AV servant à régler la valeur de l'intervalle AV ou la dépendance fonctionnelle entre des valeurs d'intervalles AV et des valeurs de fréquences de stimulation, cet intervalle ou cette dépendance étant associé(e) en tant qu'allure pondérée de façon optimale du signal cardiaque par l'unité d'évaluation de la forme de signal.

6. Dispositif de stimulateur cardiaque selon la revendication 5 en combinaison avec la revendication 1 ou en combinaison avec les revendications 1 et 4, **caractérisé en ce que** le critère programmé de pondération concerne la valeur d'amplitude de l'onde R- et/ou la valeur absolue de la pente de la réponse VER entre les maxima de la valeur R+ et de la valeur T+.

7. Dispositif de stimulateur cardiaque selon l'une des revendications précédentes, **caractérisé en ce que** le dispositif (100B) de calcul de l'intervalle AV ou l'unité de retardement AV (103A) est conçu pour la délivrance d'une valeur de remplacement de l'intervalle AV en l'absence d'un résultat de calcul réel des intervalles AV, en particulier lors du blocage du générateur d'impulsions de stimulation (102) ou de la non détection d'un signal cardiaque provoqué.

8. Dispositif de stimulateur cardiaque selon l'une des revendications précédentes, **caractérisé par** un capteur de position et/ou d'activité (5) et une unité de commande de déroulement (105, 123) reliée à ce capteur par l'intermédiaire d'une entrée de commande et servant à commander la procédure de calibrage de l'intervalle AV en fonction du signal de sortie du capteur de position et/ou d'activité.

9. Dispositif de stimulateur cardiaque selon l'une des revendications précédentes, **caractérisé par** sa réalisation sous la forme d'un stimulateur cardiaque (100) adaptatif du point de vue rythme, comprenant des moyens (100A) pour régler, en fonction de la sollicitation, la fréquence de stimulation et des moyens (127) pour associer une valeur d'intervalle AV spécifique à au moins une quantité partielle des valeurs de fréquences de stimulation.

10. Dispositif de stimulateur cardiaque selon l'une des revendications précédentes, **caractérisé par** sa réalisation sous la forme d'un stimulateur (100) à deux chambres, comportant des moyens (103B,EA) pour délivrer des impulsions de stimulation à l'oreillette (A).

11. Dispositif de stimulateur cardiaque selon l'une des revendications précédentes, **caractérisé en ce que** le dispositif de calcul de l'intervalle AV est prévu à l'extérieur d'un stimulateur cardiaque implantable, notamment dans un appareil de programmation ou dans un appareil auxiliaire de ce dernier.

12. Dispositif de stimulateur cardiaque selon l'une des revendications 1 à 10, **caractérisé en ce que** le dispositif (100B) de calcul de l'intervalle AV est agencé en tant que partie d'un stimulateur cardiaque implantable (100).
